# EUROPEAN PATENT APPLICATION

(11) **EP 4 809 916 A1**
(43) Date of publication of application: **23.09.2026**
(21) Application number: 25165029.7
(22) Date of filing: 20.03.2025
(51) Int. Cl.: A61B 5/08, A61B 5/00, A46B 15/00

(54) **HEALTH INDICATOR DETECTION USING AN ORAL CARE DEVICE**

(71) Applicant: Koninklijke Philips N.V., 5656 AE Eindhoven (NL)
(72) Inventor: KOOIJMAN, Gerben, Eindhoven (NL); VAN DEN DUNGEN, Wilhelmus Andreas Marinus Arnoldus Maria, Eindhoven (NL)
(74) Representative: Philips Intellectual Property & Standards

(57) **Abstract**

The present disclosure provides a processing device for computing a health indicator for a user of an oral care device. The processing device obtains sensor data from a sensor system of the oral care device, including fluid sensor data measuring at least one target compound in user saliva or breath. A user-device interaction signal is obtained from the sensor data to estimate oral care device placement in the mouth. A target observation time window is determined based on the estimated device placement, excluding at least a portion of the total time the device is in the mouth. The health indicator is computed using only fluid sensor data obtained during the determined observation time window. The processing device may estimate the observation time window to end before commencement of an active oral care function period. The sensor system may include additional sensors such as motion, proximity, or pressure sensors to detect user interaction.

## Description

### FIELD OF THE INVENTION

The present invention relates generally to the field of oral care devices and more particularly to the field of health monitoring using sensors integrated in an oral care device.

### BACKGROUND OF THE INVENTION

Oral care devices have become increasingly sophisticated, incorporating various sensors and technologies to monitor and improve users' oral health. These devices often include features such as pressure sensors to prevent overbrushing, timers to ensure adequate brushing duration, and connectivity options to track brushing habits over time.

Recent advancements have led to the integration of chemical sensors in oral care devices, capable of detecting compounds in saliva or breath that may indicate certain health conditions. These sensors aim to provide users with more comprehensive information about their oral and overall health status. However, the accuracy and reliability of such measurements can be compromised by various factors inherent to the oral care routine.

One significant challenge is the presence of cleaning agents, such as toothpaste or mouthwash, used during oral care routines. These substances can interfere with sensor readings by releasing compounds that affect the detection of target molecules. Additionally, the timing of measurements relative to the oral care routine can impact the validity of the data collected, as the oral environment changes significantly before, during, and after brushing or other cleaning activities.

It has been appreciated that an approach is needed that overcomes one or more of these problems.

### SUMMARY OF THE INVENTION

The invention is defined by the claims.

An aspect of the invention is a processing device for computing a health indicator for a user of an oral care device. The oral care device is for placement in the mouth for performing a powered oral care function. The processing device comprises one or more processors. The one or more processors are configured to obtain sensor data from a sensor system comprised by the oral care device. The sensor data includes at least fluid sensor data from a fluid sensor of the sensor system. The fluid sensor is configured to measure at least one target compound within user saliva or user breath.

The one or more processors are further configured to obtain, using at least a subset of the sensor data, a user-device interaction signal indicative of user-device interaction.

The one or more processors are further configured to estimate, using the user-device interaction signal, device placement in the mouth.

The one or more processors are further configured to determine a target observation time window based on the estimated device placement. The observation time window is preferably determined to exclude at least a portion of a total time period the oral care device is placed in the mouth.

The one or more processors are further configured to compute a health indicator using fluid sensor data output by the fluid sensor. The health indicator is computed using only fluid sensor data obtained during the determined observation time window.

Embodiments of the invention are based on limiting a time window within which sampling of target compounds in saliva or breath is performed with an aim of excluding a period where contamination from cleaning substances is most prevalent.

For example, it has been the recognition of the inventors that the optimal moment for gas or liquid measurement or sampling may be at a time when the oral care device has been placed within the mouth but prior to the initiation of the powered oral care action, such as brushing. The same principle is applicable to health sensing methodologies that rely on saliva markers or intra-oral imaging, wherein contamination or obscuration of measurement can occur due to toothpaste foaming or similar effects.

According to embodiments of the present invention, an approach is proposed to determine an optimal window for measurement and to trigger or process gas sensing or sampling accordingly.

Embodiments of the invention involve detecting from the sensor data a user-device interaction signal indicative of user interaction with, or use of, the personal care device. Using a timing associated with this detected signal, the processing device is operable to determine a timing for a target observation time window from which fluid measurements acquired by the fluid sensor will be taken.

The step of estimating device mouth placement may comprise for example using the detected user-device interaction signal to estimate a timing of insertion of at least a portion of the oral care device in the user's mouth.

In some embodiments, use of the device encompasses an active period during which the powered oral care function is active, and wherein the processing device is configured to estimate an observation time window which lies outside the active period. For example, in some embodiments, use of the device encompasses an active period during which the powered oral care function is active, and a pre-active period prior to the powered oral care function being activated, and wherein the processing device is configured to estimate an observation time window which lies in the pre-active period.

This may be a time after insertion in the mouth but before activation of the powered care function (e.g. activation of the brushing motor), but may additionally or alternatively include a time period before mouth insertion. For example, compounds in exhaled breath may be detectable at a time when the device is close to the mouth but still outside the oral cavity.

In some embodiments, the processing device is configured to estimate the observation time window so as to end before commencement of the active period. For example, the target observation time window may be an estimated time window ending before commencement of an oral cleaning session by the user using the oral care device.

In some embodiments, the processing device is configured to determine the observation time window so as to start after device mouth placement. By way of example, the target observation time window may be a time window commencing after insertion of the device into the mouth, but before commencement of the powered oral care function by the user.

In some embodiments, the processing device may be configured to estimate a start time of the active period, and determine the observation time window so as to lie outside the active period. In other words, the processing device is configured to perform a timing estimation for the active period and determine at least an end time for the observation time window such that the observation time window at least ends before the start of the active period.

In some embodiments, the sensor data used to obtain the user-device interaction signal may be sensor data obtained from at least one sensor having an output responsive to device movement or responsive to changes in a property of an environment around the device. In this way, the sensor data is reflective of a user moving the device or otherwise changing a state of the device relative to the environment.

In some embodiments, the detecting the user-device interaction signal comprises detecting a predetermined feature or pattern in the sensor data, and optionally wherein the predetermined feature or pattern includes a local fluctuation in the sensor data. In other words, detecting the user-device interaction signal comprises identifying a pre-determined pattern or signature in the sensor data from the sensor system.

In some embodiments, determining the observation time window comprises applying a pre-determined relationship between a timing of the predetermined feature or pattern in the sensor data and a target timing for the observation time window. In other words, the processing device may be configured to apply a pre-defined mapping or conversion between a temporal pattern of the pre-determined pattern or signature in the sensor data and a timing for the observation time window.

In some embodiments, the user-device interaction signal may be detected from fluid sensor data obtained from the fluid sensor. This provides an inherent efficiency in that the same sensor used for computing the health indicator is also used to detect user interaction with the device. For example, the fluid sensor readings can be expected to change as the part of the device carrying the fluid sensor is moved, for example moved from outside the mouth to inside the mouth or from a first position outside the mouth to a second position outside the mouth, wherein the second position is closer to the mouth that the first position. In some embodiments, the detecting the user-device interaction signal may comprise detecting at least a local peak or spike in a measured value of the target compound, or a step change in the measured value of the target compound, or a transition in a level of the measured value of the target compound from a first level to a second value. This may be indicative of insertion in the mouth.

A further aspect of the invention is an oral care system. The oral care system comprises an oral care device comprising the aforementioned sensor system configured to generate sensor data, the sensor system comprising at least one fluid sensor configured to measure at least one target compound within user saliva or user breath. The oral care device may additionally comprise a powered oral care subsystem operable to perform a powered oral care function during use of the oral care device. The oral care system further comprises the processing device in accordance with any preceding claim. The processing device may be integrated in the oral care device, or alternatively may be separate to the oral care device.

With regards to the oral care device, embodiments of the invention are applicable to any variety of oral care device. This includes but is not limited to: powered toothbrushes, powered mouthpiece devices, oral irrigators and powered flossers.

Regarding the fluid sensor of the sensor system, this may in some embodiments be arranged to be exposed to saliva in the mouth and/or exhaled breath during use of the oral care device (for example during use of the powered oral care function). By way of example, the oral care device may include a mouth-receivable portion for insertion in the mouth during use, and wherein the fluid sensor is integrated in the mouth receivable portion. By way of example, the oral care device may be a powered toothbrush comprising a brush head for insertion in the mouth during use and wherein the fluid sensor is integrated in the brush head so that the fluid sensor is positioned in the mouth during use the device. The fluid sensor is accordingly exposed to exhaled breath and saliva during normal use of the device. By way of alternative, the oral care device may include a handle portion located outside the mouth during use and wherein the fluid sensor is carried by the handle portion with a sensing region exposed at a surface of the handle portion and arranged so that the sensing region is exposed to exhaled breath of the user during use of the device (when the mouth-receivable portion is received in the mouth). For example, the sensing area is arranged facing toward the mouth when the mouth-receivable portion is received in the mouth.

By way of an alternative, in some embodiments, the device includes a sample collection system configured to obtain a saliva or gas sample from the mouth during use and expose the sensor to the collected sample. In this set of embodiments, the fluid sensor may be arranged so as to be located outside the mouth during use of the oral care device (for example during use of the powered oral care function).

In some embodiments, the processing device may be configured to trigger acquisition of sensor data using the fluid sensor only during the observation time window, and to compute the health indicator using the acquired fluid sensor data. In other words, in this set of embodiments, the processing device is configured to control sampling of the relevant sensor data only during the period of the observation time window. In this case, potentially all of the relevant sampled sensor data may then be used to compute the relevant health indicator because the acquired sensor data corresponds only to the time period spanned by the observation time window.

By way of an alternative implementation, in some embodiments, the one or more processors of the processing device may be configured to acquire sensor data using the fluid sensor for a measurement time period, wherein the measurement time period is longer than the observation time window. In this case, the processing device may be configured to extract a sub-portion of the acquired fluid sensor data spanning the observation time window, and wherein the health indicator is computed using only the extracted sub-portion of the data. In this case, the relevant sensor data is sampled over a longer time period (referred to as the measurement time period) and a relevant temporal portion of the sampled sensor data is then retrospectively selected or extracted from the sampled sensor data.

In some embodiments, the sensor system may further comprise at least one additional sensor (additional to the fluid sensor), and wherein the user-device interaction signal is obtained using additional sensor data obtained from the at least one additional sensor.

The at least one additional sensor may include at least one sensor configured to generate an output correlated with user interaction with the oral care device. For example, the at least one additional sensor may have an output responsive to device movement or responsive to changes in a property of an environment around the device.

Various options are possible for the at least one additional sensor.

In some embodiments, an output of the at least one additional sensor may be indicative of motion of the oral care device. The at least one additional sensor may comprise a motion sensor. Optionally, the at least one additional sensor includes an inertial measurement unit (IMU). The at least one additional sensor may include an accelerometer.

Additionally or alternatively, the at least one additional sensor may include a proximity or distance sensor. The proximity sensor may be configured to detect distance to the user's head. The proximity sensor may be configured to detect proximity of a mouth-receivable part of the device to oral surfaces. The proximity sensor may be configured to detect proximity of a user's hand to a handle of the device.

The proximity sensor may be optical or capacitive.

Additionally or alternatively, the at least one additional sensor may include a pressure sensor arranged to sense a pressure between at least a region of a mouth-insertable portion of the device and oral surfaces within the mouth. In this set of embodiments, pressure sensor data may be used to detect insertion in the mouth, for example insertion of a brush head into the mouth in a case where the oral care device is a powered toothbrush device.

Additionally or alternatively, the at least one additional sensor may include a user control element mechanically actuable by the user and operable to generate an output signal responsive to user actuation, and wherein the user-device interaction signal is derived from the output of the user control element.

For example, the user control element may include a device power or start/stop button configured to trigger activation of the powered oral care function of the oral care device. The start/stop button may be operable by the user to start/stop the powered oral care function for example. The button press, or on/off status can thus be used to detect if oral care action has started. This might provide an indication of an ending moment for the observation window by way of one non-limiting example.

Additionally or alternatively, the at least one additional sensor may include a humidity or temperature sensor. This may be used to detect insertion in the mouth by a user based on rapid change in humidity or temperature.

In some embodiments, the at least one additional sensor includes a gas sensor arranged for sensing a gas in the mouth of the user. In some embodiments, the at least one additional sensor includes a conductivity sensor for sensing presence of saliva. In some embodiments, the at least one additional sensor includes at least one camera. In some embodiments, the at least one additional sensor includes a vibration or acoustic sensor.

A further aspect of the invention is a method for computing at least one health indicator for a user of an oral care device, wherein the oral care device is for placement in the mouth for performing a powered oral care function. The method comprises obtaining sensor data from a sensor system comprised by the oral care device, the sensor data including at least fluid sensor data from a fluid sensor of the sensor system, the fluid sensor configured to measure at least one target compound within user saliva or user breath. The method further comprises obtaining using at least a portion of the sensor data a user-device interaction signal indicative of user interaction with the personal care device. The method further comprises estimating, using the user-device interaction signal, device placement in the mouth. The method further comprises determining a target observation time window based on the estimated device placement, the observation time window determined to exclude at least a portion of a total time period the oral care device is placed in the mouth. The method further comprises computing a health indicator using fluid sensor data output by the fluid sensor. The health indicator is computed using only fluid sensor data obtained during the determined observation time window.

A further aspect of the invention is a computer program product, comprising computer program code configured, when run by a processor, to cause the processor to perform a method in accordance with any embodiment presented in this disclosure or in accordance with any claim.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of an example processing device and system in accordance with one or more embodiments of the invention;
Fig. 3 schematically illustrates timing of one example observation time window relative to timings of different phases of device operation by a user;
Fig. 4 illustrates an example embodiment of the invention wherein the oral care device takes the form of a powered toothbrush; and
Fig. 5 illustrates sample sensor data output by different possible sensors which may be comprised by the sensor system in accordance with different embodiments, illustrating how the sensor data may be used to infer features of user-device interaction.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Embodiments of the invention provide a method and system for optimizing a timing window within which sensor data is acquired for the purpose of computing a health marker or health indicator during an oral care session utilizing an oral care device. The timing may be controlled such that it falls outside of a window during which contaminants associated with the performance of the oral care action, such as toothpaste, are present or are present only in a limited extent within the oral cavity. The optimized timing may, for example, correspond to a period following the insertion of the oral care device into the mouth but prior to the commencement of a powered cleaning function of the device.

At least some embodiments may involve determining a start and stop time for a health-related measurement - preferably a gas or liquid compound measurement - within the oral cavity. Said method may comprise the detection of oral care device positioning and presence within the oral cavity based on sensor input, the initiation of gas or liquid measurement upon sensing the oral cavity environment, and the cessation of gas or liquid measurement upon detecting the commencement of brushing.

An oral care system may also be provided, comprising a sensor configured to measure a parameter correlated with a health condition of the user. The sensor may, in typical embodiments, comprise a gas sensor for detecting one or more target gas compounds present in the exhaled breath of the user and/or within the oral cavity. Alternatively, the sensor may be configured to measure compounds present in saliva. In another embodiment, the sensor may comprise an imaging sensor.

Disclosed herein are solutions for performing or triggering the measurement or sampling of gas compounds within an optimal time window, such as a time in which the oral care device has been placed in or near the mouth but prior to the initiation of the oral care action. By implementing such a timing mechanism, interfering effects caused by cleaning agents, including but not limited to toothpaste, are minimized.

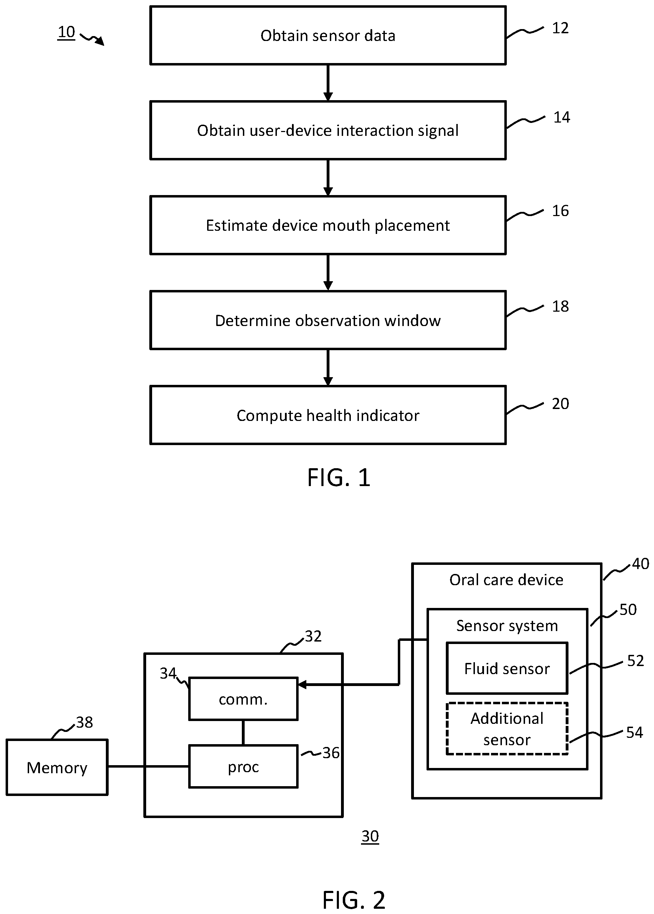

Fig. 1 outlines in block diagram form steps of an example method according to one or more embodiments. The steps will be recited in summary, before being explained further in the form of example embodiments.

The method 10 is for computing at least one health indicator for a user of an oral care device. The oral care device is for placement in the mouth for performing a powered oral care function.

The method 10 comprises obtaining 12 sensor data from a sensor system comprised by the oral care device. The sensor data includes at least fluid sensor data from a fluid sensor of the sensor system, the fluid sensor configured to measure at least one target compound within user saliva or user breath. The target compound may represent a feature or marker related to a health condition of the user. The fluid sensor may be integrated in the oral care device. Additionally or alternatively, the sensor system may include means for collecting a sample of gas and/or saliva and exposing the fluid sensor to the collected sample.

The method 10 further comprises obtaining 14, using at least a portion of the sensor data, a user-device interaction signal indicative of user interaction with the personal care device.

The method 10 further comprises estimating 16, using the user-device interaction signal, device placement in the mouth.

The method 10 further comprises determining 18 a target observation time window based on the estimated device placement. The observation time window is preferably determined so as to exclude at least a portion of a total time period the oral care device is placed in the mouth. In other words, the method comprises processing the relevant sensor data to determine a time window, and wherein that time window covers at most only a portion of the total time that the device is in the mouth. By way of one non-limiting example, this may be a time window extending between a time of placement of the oral care device in the mouth and a time at which the powered oral care function or action is started.

The method 10 further comprises computing 20 a health indicator using fluid sensor data output by the fluid sensor. The health indicator is computed using only fluid sensor data obtained during the determined observation time window.

An aim of the method is to provide an output related to the feature or marker measured by the sensor system that is substantially free from interference by cleaning agents used in the oral care routine.

As will be explained later, the computation of the health indicator using only sensor data obtained during the observation time window can be realized in different ways. One way is to trigger a measurement using the fluid sensor only during the observation time window, i.e. control sampling of the fluid sensor so as to only sample sensor data during the observation time window. Another approach is to process a sensor data set acquired using the fluid sensor so as to extract a portion of the acquired data which spans just the determined observation time window, such that the results correlate to this specific time window. In embodiments in which the sensor system includes a sample collection system, another approach is to control the means for sample collection such that a sample is only taken over the determined observation time window.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of an oral care system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises an oral care device 40.

In the illustrated example of Fig. 2, the oral care device 40 comprises a sensor system 50. The sensor system 50 includes a fluid sensor 52 configured to measure at least one target compound within user saliva or user breath. Optionally, the sensor system may include at least one additional sensor 54. In some embodiments, the obtaining 14 of the user-device interaction signal may be performed using additional sensor data obtained from the optional at least one additional sensor 54. In some embodiments, the at least one additional sensor 54 may include at least one sensor configured to generate an output correlated with user interaction with the oral care device. For example, the at least one additional sensor 54 may have an output responsive device movement or responsive to changes in a property of an environment around the device.

Although Fig. 2 shows the sensor system 50 integrated in the oral care device 40, this is not essential. In other embodiments, the sensor system 50 may be separate to the oral care device. Furthermore, although Fig. 2 shows the processing device 32 being separate to the oral care device, this is not essential. The processing device could be integrated in the oral care device in some embodiments.

In some embodiments the processing device 32 may be provided by a processing component of a mobile computing device. In other words, the processing functionality of the processing device may be performed by a mobile computing device which may be communicatively coupled with the sensor system. All or part of the functionality of the processing device may be provided by a cloud-based server in some embodiments.

With regards to the oral care device, embodiments of the invention are applicable to any variety of oral care device. This includes but is not limited to: powered toothbrushes, powered mouthpiece devices, oral irrigators and powered flossers.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

In some examples, the oral care system 30 may include a user interface configured to provide sensory feedback to the user indicating when the observation time window has started, e.g. by audio or visual means (e.g. beeping sound, light ring or haptic feedback).

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

As previously noted, in certain embodiments, the sensor system 50 may be configured to include at least one additional sensor 54, wherein the user-device interaction signal is derived through the utilization of additional sensor data obtained from the at least one additional sensor. By way of non-limiting example, the at least one additional sensor 54 may optionally comprise any one or more of the following: an on/off or start/stop button, an electronic or mechanical trigger, or a mechanical multilevel/stage switch; a capacitive proximity sensor; a motion sensor, such as an Inertial Measurement Unit (IMU); an optical proximity sensor, a pressure sensor configured to measure pressure exerted on the brush head; a touch sensor, which may include a pressure sensor or a switch located on the handle; a light intensity sensor; a motor voltage or current sensor; a gas sensor; a saliva sensor; an electrical conductivity sensor; a camera or intra-oral scanner (IOS); an acoustical sensor; a vibration sensor; a temperature sensor; or a humidity sensor.

In some embodiments, the oral care system 30 includes a fluid sensor 52 that is arranged to be exposed to saliva in the mouth and/or exhaled breath (either inside the mouth or outside the mouth) during use of the oral care device 40. In other words, the sensor is arranged to be directly exposed to saliva or breath during normal use of the device.

Alternatively, in some embodiments, the device includes a sample collection system configured to obtain a saliva or gas sample from the mouth during use and expose the sensor to the collected sample.

In some embodiments, the oral care device includes a mouth-receivable portion designed for insertion into the mouth during use, wherein the fluid sensor is integrated within the mouth-receivable portion. By way of example, the fluid sensor may be incorporated into the brush head of a toothbrush.

By way of alternative, the oral care device may include a handle portion located outside the mouth during use and wherein the fluid sensor is carried by the handle portion with a sensing region exposed at a surface of the handle portion and arranged so that the sensing region is exposed to exhaled breath of the user during use of the device (e.g. when a mouth-receivable portion is received in the mouth). For example, the sensing area is arranged facing toward the mouth when the mouth-receivable portion is received in the mouth.

In some embodiments, the processing device 32 is configured to trigger the acquisition of sensor data using the fluid sensor 52 only during an observation time window. The processing device 32 is further configured to compute a health indicator using the acquired fluid sensor data.

In further embodiments, the one or more processors of the oral care system are configured to acquire sensor data using the fluid sensor 52 over a measurement time period, wherein the measurement time period is longer than the observation time window. The processing device is further configured to extract a sub-portion of the acquired fluid sensor data that spans the observation time window. In such embodiments, the health indicator is computed using only the extracted sub-portion of the acquired fluid sensor data.

The step of computing 20 the health indicator may comprise applying a predefined computation procedure configured to infer a value of at least one health indicator based on processing of fluid sensor data generated by the fluid sensor 52. The processing device 32 may be configured to apply the computation procedure such that the health indicator is inferred based only on fluid sensor data obtained during the determined observation time window.

For example, the computing of the health indicator, may be performed by processing the sensor data obtained from the fluid sensor 52 in accordance with one or more predefined algorithms or computational models. The fluid sensor data may be indicative of the presence and/or concentration of specific target compounds in user saliva or user breath. The health indicator may be determined based on an analysis of such target compounds in combination with stored reference data, machine learning models, or mathematical formulae designed to correlate measured values with known health conditions or physiological states.

The presence and concentration of target compounds in the fluid sensor data may be determined through pattern recognition techniques, peak detection algorithms, or machine learning classifiers trained on reference spectra. Additionally or alternatively, spectroscopic or electrochemical detection methods may be employed to establish compound-specific response characteristics.

With regard to determining the health indicator, in some examples, the computed concentration of a target compound may be compared against one or more threshold values or reference ranges to determine deviations indicative of a health condition. In some examples, a weighted scoring system may be used to combine multiple compound concentrations into a composite health indicator value. In some examples, one or more pre-defined regression models, neural networks, or Bayesian inference models may be utilized to infer probabilistic health indicators based on sensor readings. In some examples, the computation may comprise application of one or more decision trees or rule-based logic systems where predefined thresholds trigger specific health classifications.

By way of non-limiting example, the following are examples of target gas compounds which may be detected in user breath and associated health indicators which may be derived using measured concentrations or levels od said gas compounds:
- Acetone: Elevated levels of acetone can indicate ketosis, which may be due to diabetes (particularly type 1 diabetes).
- Isoprene: This compound is often linked to lipid metabolism. Variations in isoprene levels can be associated with oxidative stress or changes in metabolic activity, such as during exercise.
- Nitric Oxide (NO): Increased levels can indicate airway inflammation. This is commonly monitored in conditions such as asthma.
- Methane: Elevated levels can be associated with gastrointestinal issues, such as bacterial overgrowth in the intestines or certain types of infections.
- Ethanol: Presence of ethanol in the breath can be due to the ingestion of alcoholic beverages, but in a medical context, elevated levels can be indicative of fermentation by gut microflora (auto-brewery syndrome).
- Ammonia: Increased ammonia levels can be indicative of kidney dysfunction or liver disease.
- Carbon Monoxide (CO): Elevated levels can result from smoking, environmental exposure, or hemolytic disorders.
- Hydrogen Cyanide (HCN): May be detected in smokers and can indicate exposure to cyanogenic compounds.
- Formaldehyde: Can be related to oxidative stress and potential exposure to environmental pollutants.
- Hexanal and Pentanal: These compounds may be associated with lipid peroxidation, often reflecting oxidative stress or inflammatory processes.
- Ethane and Pentane: Elevated levels can signal increased oxidative stress and lipid peroxidation, which is relevant in conditions like inflammatory diseases and cancer.
- Dimethyl Sulfide: Raised levels can be indicative of liver disease and certain metabolic conditions.

With regards to saliva, typical (bio)markers include: pH, (specific) hormones, proteins/enzymes, and/or microbiomes / bacterial metabolites.

By way of non-limiting example, the following are examples of target compounds which may be detected in user saliva and associated health indicators which may be derived using measured concentrations or levels of said compounds:.
- Cortisol: Cortisol is a hormone associated with stress. Elevated levels in saliva can indicate chronic stress, anxiety, or adrenal gland dysfunction.
- Immunoglobulin A (IgA): IgA is an antibody that plays a critical role in mucosal immunity. Low levels may suggest a weakened immune system, while elevated levels can be indicative of an active immune response to infection or disease.
- Alpha-Amylase: This enzyme is involved in the breakdown of starches. Increased levels can be associated with stress, particularly acute stress responses.
- Dehydroepiandrosterone (DHEA): DHEA is a steroid hormone that can be an indicator of adrenal function. Variations in levels might reflect issues with adrenal gland health.
- Lactate: Elevated lactate levels in saliva can be indicative of metabolic stress or conditions such as oral cancer, muscle fatigue, or systemic infections.
- Urea and Ammonia: Elevated levels in saliva can suggest kidney dysfunction or excessive protein breakdown in the body.
- Salivary Enzymes (such as lysozyme): Variations in these enzymes can be indicative of bacterial infections or inflammation in the oral cavity.
- Salivary Proteomes: Proteomic analysis of saliva can detect markers for various conditions, including oral cancers, autoimmune diseases, and systemic conditions like diabetes.
- Salivary Microbiome: Changes in the composition of bacteria and other microorganisms in saliva can indicate oral health issues, such as periodontal disease, cavities, or systemic conditions like diabetes.
- Glucose: Elevated levels of glucose in saliva can be an indicator of diabetes or impaired glucose metabolism.
- Electrolytes (such as sodium, potassium, and calcium): Abnormal levels can point to dehydration, renal issues, or metabolic disturbances.
- DNA/RNA fragments: Analysis of genetic material in saliva can offer insights into various diseases, including oral cancers and other genetic conditions.
- Hormones (such as estrogen and progesterone): Monitoring these hormones can provide information about reproductive health, menstrual cycle phases, and potential endocrine disorders.
- Inflammatory Markers (such as C-reactive protein): Presence can indicate systemic inflammation or localized inflammation in the oral cavity.

As discussed above, the processing device is configured to obtain a user-device interaction signal indicative of user interaction with the oral care device, wherein the user-device interaction signal is obtained using at least a subset of the sensor data acquired from the sensor system of the oral care device.

In some embodiments, the sensor data used to obtain the user-device interaction signal includes sensor data obtained from at least one sensor having an output responsive to device movement or responsive to changes in a property of an environment around the device. Such a sensor may include, but is not limited to, an accelerometer, a gyroscope, or an inertial measurement unit (IMU), each of which provides data indicative of motion, orientation, or changes in positioning of the oral care device. Alternatively, or in addition, the sensor may be an environmental sensor capable of detecting variations in external conditions such as temperature, humidity, or ambient light, where such variations correlate with user interaction events.

The user-device interaction signal may be obtained by detecting a predetermined feature or pattern within the sensor data. In some embodiments, the predetermined feature or pattern includes a local fluctuation in the sensor data, which may be indicative of the oral care device being placed in or removed from the oral cavity. The fluctuation may be detected based on an analysis of temporal variations in the sensor data, wherein specific thresholds or pattern recognition techniques are applied to identify characteristic changes associated with user interaction.

In further embodiments, the determination of an observation time window for assessing a health indicator may be based on a predetermined relationship between the timing of the detected predetermined feature or pattern in the sensor data and a target timing for the observation time window. For example, upon detection of a local fluctuation indicative of the oral care device being inserted into the mouth, the processing device may define a subsequent observation window during which fluid sensor measurements are analyzed for computing the health indicator.

Additionally, in some embodiments, the user-device interaction signal is detected from fluid sensor data obtained from the fluid sensor. The detection may involve identifying at least a local peak or spike in a measured value of the target compound, or a step change in the measured value of the target compound, or a transition in a level of the measured value of the target compound from a first level to a second value. The local peak or spike, or step change, or the transition in a level of the measured value may be indicative of an event such as the initial insertion of the oral care device into the mouth. For instance, an abrupt increase in the concentration of a particular target compound, such as volatile sulfur compounds (VSCs) or ammonia, may serve as an indicator of exposure to user breath or saliva, thereby confirming a user interaction event.

In certain embodiments, the processing device 32 is configured to estimate oral care device placement in the mouth using the obtained user-device interaction signal. The estimation of device mouth placement may comprise analyzing the detected user-device interaction signal to estimate a timing of insertion of at least a portion of the oral care device into the user's mouth. The timing estimation may be derived from temporal characteristics of the user-device interaction signal, such as the occurrence of a sudden fluctuation, peak, or transition in sensor data that corresponds to an insertion event.

In some embodiments, the estimation of device placement in the mouth may be based on sensor data from motion-responsive sensors, wherein an abrupt change in acceleration or orientation is indicative of movement associated with bringing the device into the oral cavity. For example, a sequence of detected motion patterns that corresponds to a known usage behavior-such as raising the device towards the face followed by a temporary stabilization-may be utilized to determine when insertion occurs.

In some embodiments, sensor data from the fluid sensor 52 may be used in estimating device placement in the mouth. An increase in the concentration of a target compound detected by the fluid sensor may serve as an additional indicator of the device's entry into the oral cavity.

The processing device may apply threshold-based detection methods or pattern recognition techniques to identify sensor signal changes and correlate them with user-device interaction signals such as mouth placement timing.

In some embodiments, the powered toothbrush may be configured such that the fluid sensor is arranged outside the mouth during use, while a sample collection system is provided to obtain a sample of saliva and/or exhaled gas from the oral cavity and expose the fluid sensor to the collected sample. The sample collection system may comprise one or more fluid channels, conduits, or capillary structures in fluid communication with the oral cavity during use of the device.

The sample collection system may be integrated within a handle portion of the powered toothbrush and may include an intake opening configured to be positioned proximate to the user's mouth during operation of the device. The intake opening may be located on an external surface of the handle or may be formed within an extension element protruding from the handle toward the oral cavity.

As discussed above, the processing device 32 is configured to determine 18 a target observation time window based on the estimated device placement, and wherein the observation time window may be determined to exclude at least a portion of a total time period the oral care device 40 is placed in the mouth.

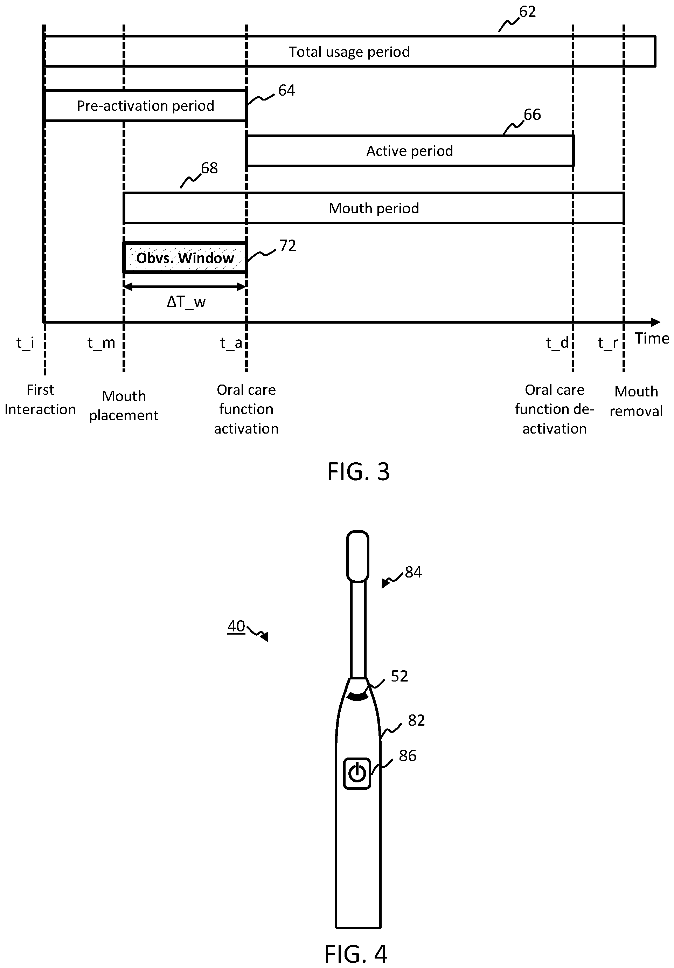

This feature will now be explained in more detail with reference to Fig. 3. Fig. 3, which schematically illustrates timing of one example observation time window relative to timings of different phases of device operation by a user.

Use of the oral care device by a user typically comprises pickup of the device, placement of the device in the mouth and subsequent activation of a powered oral care function.

A total device usage period 62 may be defined which spans a total period for which a user is interacting with the device, starting at a time of first user interaction with the device (denoted t_i). A moment of first user interaction with the device may for example correspond to a moment of device pickup.

Fig. 3 denotes a series of specific time points relevant to the phases of use of the device. These include the time of first user interaction with the device, t_i, the timing of placement of the device in the mouth, t_m, the time of activation of the powered oral care function. t_a, the time of deactivation of the powered oral care function. t_d (either manually or automatically), and the time of removal of the device from the mouth t_r.

The period during which the powered oral care function is active may be denoted as an active period 66. A time period prior to activation of the oral care function, following the user's initial interaction with the device, may be designated as a pre-activation period 64.

Furthermore, a separate time period, referred to as the mouth placement period 68, denotes the duration for which the oral care device remains positioned within the user's mouth. The mouth placement period 68 overlaps partially with the pre-activation period 64 and overlaps fully with the active period 66 (in the sense that the active period starts and ends within the mouth placement period).

An example timing of an observation time window 72 is indicated in Fig. 3.

In some embodiments, the processing device is configured to estimate an observation time window 72 that lies outside the active period 66. For example, the target observation time window 72 may be determined so as to end before the commencement of the active period 66, ensuring that it occurs prior to activation of the powered oral care function, e.g. an oral cleaning function. This configuration allows for acquisition of the sensor data during a time before significant contamination by substances used during the powered oral care function such as toothpaste.

In some embodiments, the processing device is further configured to estimate an observation time window 72 that lies within the pre-active period 64.

The observation time window 72 may be determined to start after device mouth placement. t_m, thus allowing for acquisition of sensor data following the initial insertion of the oral care device into the mouth but before activation of the powered oral care function.

By way of example, estimating a timing, t_m, of device mouth placement may be achieved through detection of a user-device interaction signal indicative of the insertion of at least a portion of the oral care device into the user's mouth. The observation time window 72 may then be set as an estimated time window commencing after insertion of the at least one fluid sensor in the mouth.

In some embodiments, the processing device may be configured to estimate the start time, t_a, of the active period 66 and to determine the observation time window 72 so as to lie outside the active period 66.

Fig. 3 shows one example observation time window 72 which lies within the mouth placement period 68, but lies outside the active period 66, lying instead within the pre-activation period 64. The observation window start time and end time are both within the mouth placement period 68 (after a time t_m of device placement in the mouth), but prior to a time t_a of activation of the powered oral care function, i.e. prior to start of the active period 66. However, other options are possible. In all cases, preferably, the observation window 72 does not overlap with the active period 66 during which the powered oral care function is active, and preferably ends before this active period 66 starts, e.g. does not occur after the end t_d of the active period. The observation window may span part of the period prior to the mouth placement period 68 so that fluid sensor data is acquired wholly or partly before the time t_m at which the device is placed in the mouth. For example, exhaled breath can be measured using a gas sensor even while the oral care device is outside of the mouth. In cases where the observation window 72 starts after mouth placement, it may span only a portion of the period that the device is placed in the mouth and prior to activation t_a of the powered oral care function (rather than the whole of this period, as is indicated illustratively in Fig. 3).

As noted above, in some embodiments, the sensor system 50 further comprises at least one additional sensor 54, wherein the user-device interaction signal is obtained using additional sensor data obtained from the at least one additional sensor. Various example embodiments of the invention will now be discussed, each employing a different type of additional sensor data for determining the observation time window.

In at least one set of embodiments, the at least one additional sensor 54 includes a proximity sensor. The proximity sensor may be optical or capacitive.

A capacitive proximity sensor may be configured to sense when a user is within a threshold proximity to the oral care device, and/or to detect when a user is holding the device, e.g. when a user is holding a handle of the device. For example, capacitive proximity sensor may be integrated in a handle of the oral care device 40 and may detect when a user picks up the device for use. In this case, a user-device interaction signal can be obtained at least in part from an output of the capacitive proximity sensor comprising a user pickup signal indicative of a moment of device pickup by the user, which would be reflected in a spike or step change in an output of the proximity sensor. Although the proximity sensor output alone cannot directly detect a moment that the oral care device is placed in the mouth, the processing device may use the user-device interaction signal extracted from the sensor (i.e. device pickup signal) in combination with a pre-determined typical interval time between device pickup and device placement in the mouth to infer a time, t_m, of device mouth placement. In other words, there is a pre-determined relationship between a timing of a predetermined feature or pattern in the sensor data (in the form of a device pickup signal detectable in the proximity sensor output as a spike or step change) and an estimated timing for the device mouth placement.

The time interval between device pickup and mouth placement or between device pickup and activation of the powered oral care function may either be an assumed pre-defined value, e.g. based on a population average value, or might be learned per individual user using proximity sensor data in combination with detection of timings of activation of the powered oral care function by the individual user using the power button.

The observation time window may then be determined based on the estimate timing of mouth placement, for example it may be determined to start a certain amount of time after mouth placement, which may correspond to a pre-defined time interval following detection of device pickup from the proximity sensor output. In some embodiments, the processing device 32 may be configured to detect activation by the user of the powered oral care function, via actuation of the user control element, and may be configured to determine an end time of the observation time window at least in part based on this timing. For example, the observation time window is determined so as to end at or before activation of the powered oral care function by the user. Thus, in some embodiments, a start time of the observation time window may be determined based on the estimated time of device mouth placement (inferred from the proximity sensor data) and the end time of the observation time window may be determined at least in part based on detected timing of activation of the powered oral care function by the user, via detection of actuation of the user control element.

In some embodiments, if a time interval between detection of device pickup and activation of the powered oral care function exceeds a threshold, the processing device may be configured to infer that the device mouth placement estimation may be flawed and the fluid sensor measurement may be discarded.

In some embodiments, the observation time window may be determined to be a set duration of time following estimated timing of device placement in the mouth and preceding detected activation of the powered oral care function by the user.

In some embodiments, the processing device 32 may be configured to activate sampling of sensor data from the fluid sensor 52 immediately following detection of device pickup in the capacitive sensor output and to terminate device sensor data sampling responsive to activation of the powered oral care function by the user. This results in a sensor dataset spanning a certain measurement window. Subsequently a sub-portion of this dataset acquired for the measurement time window may be extracted, the sub-portion spanning a determined time window for the observation time window. For example, this may be a time window after mouth placement and before oral care function activation. In some embodiments, it may be determined as a final portion of the measurement time window, e.g. a final section of the measurement time window of a pre-defined window duration.

Alternatively to the capacitive proximity sensor, a touch or grip sensor on the handle may be used, and the same principles outlined above applied to this sensor data. An output of such as sensor would correspond to a user-device interaction signal indicative of a user contacting or holding the device.

Additionally or alternatively, in some embodiments, an output of the at least one additional sensor 54 may be indicative of motion of the oral care device. For example, in some embodiments, the at least one additional sensor 54 includes an inertial measurement unit (IMU) integrated in the oral care device 40.

An inertial measurement unit (IMU) typically measures acceleration, angular velocity, and magnetic field in 3 orthogonal directions. When the oral care device is not moving, no acceleration (other than gravity) and no angular velocity (when drift corrected) is measured. When the device is picked up, the motion of the device results in changes in the IMU signal. Thus, from the output of the IMU, a user-device interaction signal can be obtained indicative of user pickup and/or other movement of the device.

By way of example, a user picking up the device will result in measurement of a positive acceleration (increasing velocity) in one direction, and subsequent placement of the device in the mouth results in a subsequent negative acceleration (decreasing velocity). Thus, a user-device interaction signal indicative of device pickup and placement in the mouth can be obtained from the IMU sensor output by detecting a pre-determined pattern of positive acceleration followed by negative acceleration.

In a similar way, pre-determined signal features or fingerprints or signatures in the IMU sensor may be detectable, each associated with one of a set of pre-determined user-device interaction events. For example, these may include signal features corresponding to device pick-up, device mouth placement, toothpaste application, and/or device removal from the mouth, among others.

Hence, a user-device interaction signal indicative of mouth placement of the device can be obtained from the IMU signal by detecting a predetermined signal feature in the form of a period of deceleration following a period of acceleration, where the acceleration corresponds to moving the device toward the mouth and deceleration corresponds to stopping moving the device once it is in the mouth. The observation time window may then be determined based on the detected timing of the mouth placement, for example as a window of time starting after device mouth placement. As discussed previously, an end time of the observation time window may optionally be determined based on a detected timing of actuation of a user control button by a user to trigger activation of the powered oral care function.

In some embodiments, the at least one additional sensor 54 may include an optical proximity sensor. The optical proximity sensor may be integrated in a handle of the oral care device 40. An output of such an optical proximity sensor may be indicative of a distance to the user's face. In particular, an output of the sensor may be null during times when the device is still or when being held or picked up by the user but positioned away from the face. However, the sensor may be configured to generate a detectable signal when the device is placed within a threshold proximity to the mouth. Thus, a user-device interaction signal may be obtained from an output of such an optical proximity sensor which is indicative of movement of the device to a position within a threshold proximity of the mouth, which signal may be detectable as a spike or step change in an output of the optical proximity sensor. From this signal, a timing of placement of the device in the mouth can be estimated. For example, it may be estimated as occurring a pre-defined time delay following detection of an uptick in the output of the proximity sensor, or it may be taken as corresponding to a pre-determined threshold signal level in the output of the optical proximity sensor. A start time of the observation time window may then be determined as being a time after estimated time of device placement in the mouth. Optionally, an end time of the observation time window may be set a pre-defined time period following the start time, or may be set based on a timing of detection of activation of the powered oral care function by the user.

Additionally or alternatively to the options outlined above, in some embodiments, the at least one additional sensor 54 may include a pressure sensor arranged to sense a pressure between at least a region of a mouth-insertable portion of the oral care device 40 and oral surfaces within the mouth. For example, when the oral care device is a powered toothbrush, the mouth-insertable portion may be a brush-head of the device. The pressure sensor may be arranged to sense a pressure or force exerted on the brush head.

In this embodiment, pressure sensor data can be used to detect insertion in the mouth, for example insertion of a brush head into the mouth, based on detecting a pattern or feature in the pressure signal.

No force is detected when the oral care device is still or when it is being picked up and moved by the user. However, when toothpaste is applied, a certain force may be detected, and subsequently as soon as the brush is placed in the mouth and pressed against teeth also a force will be detected. Each of these events is associated with a detectable feature or signature or fingerprint in the pressure output signal of the pressure signal. Accordingly, a user-device interaction signal can be obtained from the output of the pressure sensor which is indicative at least of a timing of placement of the device in the mouth, this taking the form of a spike or fluctuation or uptick in the pressure signal output due to pressure against the brush head by the teeth. Thus, a timing of mouth placement can be estimated. This can then be used to determine a timing of the observation time window in a similar way as has been described previously. For example, a start time of the observation time window may be determined as being a time after estimated time of device placement in the mouth. Optionally, an end time of the observation time window may be set a pre-defined time period following the start time, or may be set based on a timing of detection of activation of the powered oral care function by the user.

In some embodiments, the at least one additional sensor 54 may include a humidity or temperature sensor.

A humidity sensor can be used to detect entering of the oral cavity (i.e. placement of the device in the mouth) or coming close to the oral cavity, since relative humidity will typically increase, up to 100%.

Similarly, a temperature sensor can be used, as temperature in the oral cavity is close to 37 degrees Celsius, which is typically well above the ambient temperature.

Hence, in some embodiments, a user-device interaction signal can be obtained from a humidity sensor or a temperature sensor which is indicative of mouth placement of the device, based on a spike or uptick in the relevant sensor output, and wherein this can be used to estimate a timing of device mouth placement.

In some embodiments, the at least one additional sensor may comprise an additional gas sensor. Changes in the output of the gas sensor can be used to infer timing of mouth placement of the device. For example, CO₂ can be expected to have a markedly increased concentration inside the mouth compared to outside. Thus, a user-device interaction signal indicative of device mouth placement can be obtained from an output of such a gas sensor.

In some embodiments, a user-device interaction signal can be obtained from an output of the primary fluid sensor 52 comprised by the sensor system 50. For example, when moving a mouth-receivable portion of the oral care device 40 from outside the mouth to inside the mouth, the fluid sensor reading can be expected to increase. Thus, placement of the device in the mouth can be estimated from an output of the fluid sensor 52 comprised by the device. In addition, one or more further stages of the usage cycle of the device are detectable from the fluid sensor. For example, activation of the powered oral care function may be detectable in the fluid sensor output, since this may cause rapid contamination, with the release of gasses, which results in a characteristic fluctuation or spike or change in the output of the fluid sensor.

If a separate additional gas sensor is used, then it may be configured to detect a different gas compound than the primary gas sensor, for example a compound for which concentration in the oral cavity is usually detectably different than the ambient (e.g. CO₂).

In some embodiments, the at least one additional sensor 54 may include a light sensor. For example the light sensor may be integrated in a mouth-receivable portion of the oral care device, e.g. the brush head of the oral care device. A user-device interaction signal indicative of placement of the device in the mouth can be obtained from the light sensor, in the form of a step down in the light signal detected by the light sensor, indicative of moving the device from outside to inside the mouth.

Instead of integrating the light sensor in the brush head, the sensor itself may be integrated in a handle of the device, and a light guide may be provided optically coupling a light-sensitive area of the light sensor to a light sampling region integrated in the brush head. When entering the oral cavity with the brush head, measured light intensity will drop. This can thus be used to determine the timing of mouth placement. From this, the timing of the observation time window can be determined in the manner already discussed above.

In some embodiments, the at least one additional sensor 54 may include an electrical conductivity sensor configured to detect presence of saliva in an environment of the sensor. A user device interaction signal indicative of device mouth placement can be obtained from an output of the electrical conductivity sensor if integrated in a mouth-receivable portion of the device such as the brush head. When the sensor is placed in the mouth, detected electrical conductivity will increase due to the presence of saliva. Additionally or alternatively, a saliva sensor sensitive to one or more target compounds in saliva, and characteristically present in saliva, may be used.

In some embodiments, the at least one additional sensor 54 may include an (intra-oral) camera. A user-device interaction signal can be obtained from image or video analysis applied to the output image or video data from the camera. This can be employed to detect the entering of the oral cavity and/or the start of brushing / foaming.

In some embodiments, the at least one additional sensor may include a vibration and/or acoustic sensor. Vibrations and sound can be used to classify the different states of the oral care routine. For example, activation of a motor used as part of the powered oral care function can be detected. However, additionally or alternatively, placement of the mouth-receivable portion of the device in the mouth is also detectable, and/or tooth scrubbing action.

Any one or a combination of the above-mentioned sensors may be included in the oral care system or device in accordance with various embodiments. Furthermore, in some embodiments, a user-device interaction signal may be extracted from the motor or system electrical voltage to determine the different states of the system including the timing of activation of the powered oral care action.

Fig. 4 schematically illustrates an example embodiment of the invention in which the oral care device 40 is a powered toothbrush. The toothbrush comprises a handle 82 and a brush head 84.

In this example, the fluid sensor 52 configured to measure at least one target compound is a gas sensor configured to measure at least one target compound within exhaled user breath. The gas sensor 52 is integrated in the handle 82 of the powered toothbrush, such that gases in exhaled breath are measured when the toothbrush is placed in the mouth.

Alternatively, the sensor 52 may be integrated in the brush head 84, such that gases in the oral cavity are directly measured. Alternatively, a guide may be provided to guide gases from the oral cavity towards a sensor in the handle.

When toothpaste is used, it can be expected that gas compounds are released from it in the oral cavity, especially once the user commences brushing and the toothpaste begins to foam. These compounds can also contaminate exhaled breath, and thus interfere with target gas compound measurement.

Therefore, in an ideal case, the target gas measurement (or target gas sampling) is performed in an observation time window between a moment of placement of the toothbrush head in the mouth (or a moment when the toothbrush head is close to the mouth) and a moment when the powered oral care action (powered brushing in this case) commences. This time window can be determined by using one or more of the additional sensor modalities discussed above integrated in the oral care device, or based on use of the fluid sensor 52 itself, to estimate a timing of placement of the brush head in the mouth, in the manner discussed above. From this, an optimal timing for the observation time window can be determined.

Some example implementations particularly applicable for a toothbrush embodiment will now be discussed.

As discussed above, the start/stop button 86 is controllable by the user to start/stop the powered oral care function, i.e. the powered brushing action in this case. The button press, or on/off status, can be used to determine a start time of the powered oral care functionality. This can be used to define an end time for the observation time window (i.e. prior to the starting of the powered oral care action), and wherein a start time for the observation time window can be determined based on the estimated time of placement of the device in the mouth or within a threshold proximity of the mouth.

Additionally or alternatively, a proximity sensor may be integrated in the handle 82 for detecting when a user is holding the device. An output from this sensor can be used to obtain a user-device interaction signal indicative of user pick up and holding of the device, and this can be used to indirectly estimate a time of placement in the mouth, e.g. based on an assumed predetermined time delay between pickup and mouth placement.

Additionally or alternatively, an IMU motion sensor may be integrated in a handle 82 of the oral care device. This may be utilized during operation for inertial position detection of the brush head relative to the teeth. However, the same sensor can be used to detect pickup of the device or movement of the device into the mouth. The means for implementing this have already been described above and so will not be repeated here.

Additionally or alternatively, an optical proximity sensor may be integrated in the handle 82 of the device. This may be configured to detect distance to the user's head. This may be used in the manner already discussed above.

Additionally or alternatively, a pressure sensor may be integrated in the oral care device for measuring a pressure or force exerted on the brush head 84. In the manner already discussed above, an output from the pressure sensor may be used to estimate a timing of placement of the device in the mouth. This can be used to determine a start time for the observation time window. The output from the pressure sensor can also be used to estimate a start time of the powered cleaning function. For example, when this begins, the user may apply the brush head against the teeth or begin moving the brush head over the teeth which results in a characteristic and detectable pattern in the pressure sensor output. Thus, a timing of a start of the powered oral care function can be estimated also from the pressure sensor output and this can be used to determine an end time for the observation time window in accordance with one or more embodiments.

As discussed above, using any one or more of the above sensing modalities, a timing for placement of the brush head (or other mouth-insertable portion of an oral care device) can be estimated, and from this a start and end of an observation time window can be determined.

A start time of the observation time window coinciding with the toothbrush placement in the mouth may be advantageous in case environmental gases can influence the measurement. An ending time for the observation time window prior to commencement of the powered oral care function (i.e. when brushing has not yet started) is also beneficial to ensure measurement or sampling is performed before contamination by foaming toothpaste.

It will be recognized that the observation time window may have a pre-defined duration. In this case, determining the observation time window may involve simply determining a start time of the observation time window. In some embodiments, the observation time window may be set to start immediately following estimated time of mouth placement of the oral care device. In this case, just determining the starting moment would suffice (as it can be assumed that brushing has not started at the end of the pre-determined duration of the gas measurement/sampling). Likewise, when the environment is not significantly influencing the measurement, just determining an end time for the observation time window may suffice if a sufficiently fast measurement or sampling stop can be achieved.

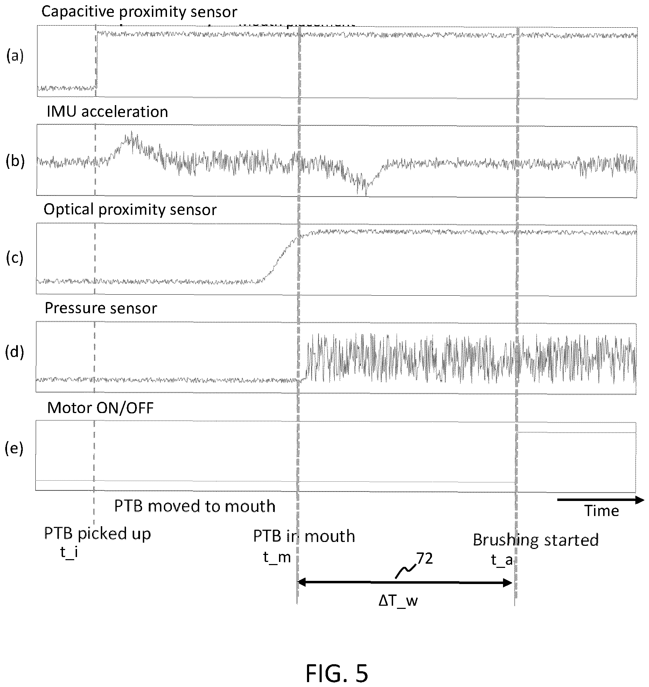

Fig. 5 illustrates example sensor signals output from a number of different sensor modalities which may be integrated in an oral care device 40 and may be used to obtain the user-device interaction signal, and, in turn, to estimate the oral care device placement in the mouth. Indicated in Fig. 5 are: the time the oral care device is picked up by the user (t_i), the time that the oral care device is placed in the mouth (t_m), and a time that the powered oral care function is activated, e.g. powered brushing is activated (t_a).

Fig. 5(a) illustrates an output of a capacitive proximity sensor integrated in the handle of the oral care device for sensing user pickup and holding of the device. As indicated, at the time, t_i, that the user picks up the device, the output of the proximity sensor exhibits a step change in the sensor signal level. This step change in the proximity sensor signal represents a user-device interaction signal indicative of user pick-up of the device.

Fig. 5(b) shows an acceleration signal output of an IMU sensor integrated in an example oral care device. Following a time, t_i, of pickup of the device, there is a marked increase in acceleration, this corresponding to the upward motion of the brush as it is picked up. This is typically the acceleration component along the length axis of the oral care device (e.g. toothbrush), after correcting for gravity. Following pickup time, t_i, the acceleration peaks and falls back to close to zero. Following a time, t_m, of placement of the device in the mouth, there is marked period of negative acceleration (deceleration) as the user brings the device to rest in the mouth. The acceleration then returns to a value close to zero. Thus, a user-device interaction signal can be obtained from the acceleration sensor output of the IMU sensor indicative of user pickup and of device placement in the mouth, wherein pickup is associated with a local positive peak in acceleration and mouth placement is associated with a local negative peak (trough) in the signal.

Fig 5(c) shows an output of an example optical proximity sensor integrated in the handle for detecting proximity of the handle to the user's head. As illustrated, as the device is moved into the mouth, an output of the optical proximity sensor exhibits a step up from a first (lower) level to a second (higher) level. Thus, a user-device interaction signal can be obtained from the output of the optical proximity sensor indicative of device placement in the mouth, wherein mouth placement is associated with a step up in the sensor signal level.

Fig. 5(d) shows an output of an example pressure sensor integrated in the oral care device and arranged to sense a pressure or force between the brush head and oral surfaces. As illustrated, the brush pressure sensor registers zero prior to a time, t_m, of placement of the brush head in the mouth. Following a time, t_m, of device mouth placement, the pressure sensor output exhibits oscillatory motion, reflective of back-and-forth motion of the brush head, e.g. scrubbing, within the mouth. Thus, a user-device interaction signal can be obtained from the output of the pressure sensor indicative of device placement in the mouth, wherein mouth placement is associated with a time of change of the signal level from a (consistent) zero pressure level to a (consistent) fluctuating or oscillating non-zero value.

Fig. 5(e) shows a motor on/off indicator signal. This signal exhibits a step change at a time, t_a, of activation of the powered oral care function, e.g. activation of the brush oscillation motor. Thus, this signal can be used as an indicator of a time, t_a, of activation of the powered oral care function. This may thus optionally be used in determining an end time for the observation time window.

A timing for one example observation time window 72, having a time duration designated as ΔT_w, is indicated in Fig. 5. In this example, the observation time window is determined so as to have a start time just after a time, t_m, of placement of the device in the mouth and an end time just prior to commencement of the powered oral care function, e.g. start of the powered brushing in the illustrated example.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processing device (32) for computing a health indicator for a user of an oral care device (40), wherein the oral care device is for placement in the mouth for performing a powered oral care function, the processing device comprising one or more processors (36) configured to:
obtain (12) sensor data from a sensor system (50) comprised by the oral care device (40), the sensor data including at least fluid sensor data from a fluid sensor (52) of the sensor system, the fluid sensor configured to measure at least one target compound within user saliva or user breath;
obtain (14) using at least a subset of the sensor data a user-device interaction signal indicative of user interaction with the oral care device;
estimate (16), using the user-device interaction signal, oral care device placement in the mouth;
determine (18) a target observation time window based on the estimated device placement, the observation time window determined to exclude at least a portion of a total time period the oral care device is placed in the mouth; and
compute (20) a health indicator using fluid sensor data output by the fluid sensor,
wherein the health indicator is computed using only fluid sensor data obtained during the determined observation time window.

2. The processing device of claim 1, wherein use of the device encompasses an active period during which the powered oral care function is active, and wherein the processing device is configured to estimate an observation time window which lies outside the active period.

3. The processing device of claim 1 or 2, wherein use of the device encompasses an active period during which the powered oral care function is active, and a pre-active period prior to the powered oral care function being activated, and wherein the processing device is configured to estimate an observation time window which lies in the pre-active period.

4. The processing device of claim 2 or 3, wherein the processing device is configured to estimate the observation time window so as to end before commencement of the active period.

5. The processing device of any of claims 2-4, wherein the processing device is configured to determine the observation time window so as to start after device mouth placement.

6. The processing device of any preceding claim, wherein the sensor data used to obtain the user-device interaction signal is sensor data obtained from at least one sensor having an output responsive to oral care device movement or responsive to changes in a property of an environment around the oral care device.

7. The processing device of any preceding claim,
wherein the detecting the user-device interaction signal comprises detecting a predetermined feature or pattern in the sensor data, and optionally wherein the predetermined feature or pattern includes a local fluctuation in the sensor data, and
optionally wherein the determining the observation time window comprises applying a pre-determined relationship between a timing of the predetermined feature or pattern in the sensor data and a target timing for the observation time window.

8. An oral care system (30), comprising:
an oral care device (40) comprising the sensor system (50) configured to generate sensor data, the sensor system comprising at least one fluid sensor (52) configured to measure at least one target compound within user saliva or user breath; and
the processing device (32) in accordance with any preceding claim.

9. The oral care system of claim 8,
wherein the fluid sensor is arranged to be exposed to saliva in the mouth and/or exhaled breath during use of the oral care device; or
wherein the device includes a sample collection system configured to obtain a saliva or breath sample during use and expose the sensor to the collected sample.

10. The oral care system of claim 8 or 9, wherein the processing device is configured to:
trigger acquisition of sensor data using the fluid sensor only during the observation time window, and compute the health indicator using the acquired fluid sensor data; or
acquire sensor data using the fluid sensor for a measurement time period, wherein the measurement time period is longer than the observation time window, and wherein the processing device is configured to extract a sub-portion of the acquired fluid sensor data spanning the observation time window, and wherein the health indicator is computed using only the extracted sub-portion of the data.

11. The oral care system of any of claims 8-10,
wherein the sensor system (50) further comprises at least one additional sensor (54), wherein the user-device interaction signal is obtained using additional sensor data obtained from the at least one additional sensor, and
optionally wherein the at least one additional sensor includes at least one sensor configured to generate an output correlated with user interaction with the oral care device.

12. The oral care system of claim 11, wherein an output of the at least one additional sensor is indicative of motion of the oral care device, and optionally wherein the at least one additional sensor includes an inertial measurement unit, IMU.

13. The oral care system of claim 11 or 12, wherein the at least one additional sensor includes any one or more of:
a proximity or distance sensor;
a pressure sensor arranged to sense a pressure between at least a region of a mouth-insertable portion of the oral care device and oral surfaces within the mouth; and
a humidity and/or temperature sensor.

14. A method (10) for computing at least one health indicator for a user of an oral care device, wherein the oral care device is for placement in the mouth for performing a powered oral care function, the method comprising:
obtaining (12) sensor data from a sensor system comprised by the oral care device, the sensor data including at least fluid sensor data from a fluid sensor of the sensor system, the fluid sensor configured to measure at least one target compound within user saliva or user breath;
obtaining (14) using at least a portion of the sensor data a user-device interaction signal indicative of user interaction with the personal care device;
estimating (16), using the user-device interaction signal, device placement in the mouth;
determining (18) a target observation time window based on the estimated device placement, the observation time window determined to exclude at least a portion of a total time period the oral care device is placed in the mouth; and
computing (20) a health indicator using fluid sensor data output by the fluid sensor;
wherein the health indicator is computed using only fluid sensor data obtained during the determined observation time window.

15. A computer program product, comprising computer program code configured, when run by a processor, to cause the processor to perform a method in accordance with claim 14.
